# DEMANDE DE BREVET EUROPEEN

(11) **EP 0 662 342 A1**
(43) Date de publication de la demande: **12.07.1995**
(21) Numéro de dépôt: 94403040.2
(22) Date de dépôt: 28.12.1994
(51) Int. Cl.: B01J 23/70, B01J 27/053, C07C 2/52

(54) **Nouveau procédé de régénération d'un catalyseur impur à base d'acide sulfurique déposé sur silice**

(30) Priorité: 06.01.1994 FR 9400144
(71) Demandeur: INSTITUT FRANCAIS DU PETROLE, F-92502 Rueil-Malmaison (FR)
(72) Inventeur: Joly, Jean-François, F-75003 Paris (FR); Benazzi, Eric, F-78360 Montesson (FR); Chaigne, Frédéric, F-85230 Bauvoir sur Mer (FR); Bernhard, Jean-Yves, F-91540 Mennecy (FR); Viltard, Jean-Charles, F-26000 Valence (FR)
(74) Mandataire: Andreeff, François

(57) **Abrégé**

L'invention concerne un procédé de régénération d'un catalyseur impur à base d'acide sulfurique déposé sur silice.

Elle est caractérisée en ce que le catalyseur est soumis à au moins une calcination en vue d'éliminer les matières organiques hydrocarbonées que contenait ce catalyseur notamment à l'issue de son utilisation dans les réactions d'alkylation aliphatique.

## Description

La présente invention concerne un nouveau procédé d'élimination des matières organiques hydrocarbonées contenues dans un catalyseur à base de silice et d'acide sulfurique impur, parce que contaminé au cours des réactions chimiques pour lesquelles le catalyseur a été utilisé. Elle concerne également le traitement de l'effluent gazeux résultant de l'élimination des matières organiques hydrocarbonées.

La présente invention concerne en particulier un procédé de traitement d'un catalyseur usé à base de silice et d'acide sulfurique provenant de la réaction d'alkylation catalytique d'isobutane et/ou d'isopentane au moyen d'une oléfine, qui permet d'obtenir au moins un produit par exemple dans le groupe constitué par les diméthylbutanes, les triméthylpentanes, les triméthylhexanes et les triméthylheptanes.

De nombreux procédés chimiques et pétrochimiques utilisent l'acide sulfurique comme catalyseur. Cet acide est en règle générale recyclé aussi longtemps que sa teneur en impuretés, notamment organiques, le permet. On opère alors une purge d'un acide sulfurique relativement chargé en matières organiques qui est envoyé dans une usine de retraitement. Le seul procédé industriel de traitement de ces rejets d'acide sulfurique est un procédé au cours duquel l'acide est transformé en dioxyde de soufre SO₂ par combustion, puis celui-ci est transformé en anhydride sulfurique (SO₃) qui est ensuite lui même retransformé en acide sulfurique par absorption dans l'eau. Parmi les procédés producteur d'acide sulfurique impur, souvent dénommé boues sulfuriques, on peut citer à titre d'exemple le procédé de synthèse d'alcools à partir d'hydrocarbures éthyléniques (en particulier la synthèse d'éthanol à partir d'éthylène, la synthèse d'isopropanol à partir du propylène et de celle de butanol-2 à partir d'un mélange de butène-1 et de butène-2), le procédé d'alkylation de l'isobutane par des oléfines telles que le propylène ou les butènes et les procédés de purification des hydrocarbures dans les opérations de raffinage.

Ces procédés, et en particulier le procédé d'alkylation, produisent des quantités d'acide usé relativement faibles qui sont encore aujourd'hui considérées comme trop faibles pour être traitées sur place. C'est ainsi que le produit est alors expédié dans des usines de production d'acide sulfurique où il est retransformé en acide sulfurique pur qui est alors reexpédié vers l'unité d'alkylation. Très souvent, les unités de traitement des bornes sulfuriques sont éloignées du lieu de production de ces boues, ce qui entraîne de nombreux risques liés au transport, généralement routier, d'un produit aussi dangeureux et polluant que les bornes sulfuriques ou l'acide pur. Ce traitement comprend une première étape de combustion par la transformation de l'acide en SO₂. De plus, l'acide pur obtenu est le plus souvent plus cher que de l'acide frais et l'usine de retraitement n'accepte d'effectuer ce traitement que si le client fournisseur de la boue reprend cet acide pur, ce qui implique pour l'usine d'alkylation un surcoût non négligeable.

De nombreux procédés de purification d'acide usé on été décrits antérieurement. C'est ainsi que le brevet US-A-3652708 décrit une méthode de réduction de la teneur en hydrocarbures d'un acide résiduaire par traitement par un excès d'oléfine avant son envoi dans une usine de combustion, ce qui n'élimine pas complètement les problèmes mentionnés ci-avant. Le procédé décrit dans le brevet européen EP-B-52548 utilise de l'acide nitrique comme oxydant des matières organiques hydrocarbonées. Ce procédé implique le traitement des gaz formés dans une unité d'élimination des oxydes d'azote, ce qui est un inconvénient important. Il a également été décrit dans un article de Shenfel'd D.E. et al publié dans Zhurnal Prikaladnoi Khimii, Vol. 61, n°7, pp. 1550 à 1553, July 1988, un procédé de traitement d'un acide usé par décomposition de l'acide usé en deux étapes. Au cours de la première étape réalisée à une température comprise entre 50 et 270°C, il se forme un résidu solide noir ressemblant à du coke. Ce résidu, dont la masse correspond sensiblement à la teneur en carbone de l'acide usé de départ, est ensuite oxydé en présence d'un courant d'air à une température supérieure à 400°C. Au cours de la première étape, la conversion des matières premières organiques hydrocarbonées présentes dans l'acide usé de départ, mesurée à partir de la quantité d'oxydes de carbone formés par réaction avec l'acide sulfurique est d'environ 12%. Le procédé décrit dans ce document présente l'inconvénient majeur de produire un résidu carboné solide et de nécessiter une oxydation à l'air de ce résidu carboné à une température très élevée. Dans la demande de brevet français de numéro d'enregistrement national 92/02072, des agents dont le pouvoir oxydant est supérieur au pouvoir oxydant de l'acide sulfurique sont introduits dans l'acide usé à traiter, parmi ces agents oxydants sont cités les composés H₂O₂, H₂SO₅ et H₂S₂O₈. L'utilisation de ces agents rend le procédé complexe et coûteux.

Le procédé de la présente invention permet d'éliminer les problèmes liés aux techniques mises en oeuvre dans les procédés de l'art antérieur et propose une solution qui peut être mise en place facilement sur le lieu de production de la boue sulfurique provenant notamment d'une unité d'alkylation. Ce nouveau procédé est particulièrement intéressant ici pour un procédé d'alkylation de l'isobutane par les oléfines utilisant un catalyseur constitué de silice imprégnée d'acide sulfurique comme cela est décrit dans la demande de brevet français de numéro d'enregistrement national 91/13303.

Le procédé selon la présente invention concerne un procédé d'élimination des matières organiques hydrocarbonées contenues dans ce type de catalyseur à base d'acide sulfurique sur silice, ledit acide sulfurique impur comprenant généralement à ce stade d'environ 50 à environ 99,5% en poids d'acide sulfurique et au moins 0,1% en poids, exprimé en atomes de carbone, de matières organiques sous forme libre ou combinée.

Comme indiqué ci dessus, l'acide sulfurique impur imprégné sur la silice peut avoir comme origine un catalyseur d'alkylation comme décrit dans la demande de brevet français de numéro d'enregistrement national 91/13303 et qui a été purgé de l'unité d'alkylation pour être retraité.

A titre d'information, le diamètre moyen des particules de catalyseur est généralement compris entre 0,1 et 200 microns (1 micron = 10⁻⁶ m).

Le procédé de traitement de l'acide impur contenu au sein de la porosité de la silice comprend une ou deux étapes. Selon la composition chimique de l'acide sulfurique impur à traiter, il est possible de ne réaliser que la première étape.

Dans la première étape, le catalyseur constitué de silice imprégnée par de l'acide impur est calciné sous débit d'un gaz, et par exemple d'un gaz contenant de l'oxygène moléculaire, par exemple de l'air ou de l'oxygène pur, de débit compris entre 0,05 et 10 l/h/g de matière à traiter et de préférence entre 0,1 et 5 l/h/g, à une température comprise entre 100 et 400°C, et de préférence comprise entre 100 et 350°C et de manière encore plus préférée entre 170 et 330°C. La durée de ce traitement est avantageusement comprise entre quelques minutes (3 minutes par exemple) et 8 heures.

Dans une seconde étape, facultative, on calcine, sous débit d'un gaz, et par exemple d'un gaz contenant de l'oxygène moléculaire, par exemple de l'air ou de l'oxygène pur, de débit compris entre 0,05 et 10 l/h/g de matière à traiter, le solide obtenu à la fin de la première étape à une température comprise entre 400 et 600°C et de préférence comprise entre 450 et 550°C de façon à éliminer les dépôts hydrocarbonés encore présents sur la silice à la fin de la première étape.

Le traitement de calcination, première et seconde étape, conduit à la production d'une phase gazeuse comprenant les produits formés par oxydation des composés hydrocarbonés initialement présent dans l'acide sulfurique impur et par du dioxyde de soufre SO₂, et à une phase liquide condensable constituée d'acide sulfurique purifié.

Il a été découvert que d'une façon surprenante, la première étape permet de récupérer une part importante de l'acide sulfurique contenu dans la silice l'acide sulfurique est ainsi condensé des vapeurs sortant du four tubulaire dans lequel se déroule la calcination de la première étape du procédé selon l'invention. L'acide sulfurique condensé est suffisamment pur pour entrer dans la fabrication d'un catalyseur d'alkylation aliphatique, après mélange avec un oléum de façon à ce que la teneur en eau de la solution d'acide sulfurique ainsi préparée soit inférieure à environ 2% en poids et après réimprégnation sur de la silice. De plus, la silice calcinée à l'issue de la deuxième étape du procédé selon l'invention peut être de nouveau imprégnée par de l 'acide sulfurique, on obtient ainsi un nouveau catalyseur d'alkylation.

Dans le procédé selon l'invention, les gaz formés au cours de la deuxième étape de calcination et plus particulièrement au cours de la première partie de la deuxième étape ne sont, le plus souvent, pas rejetés directement dans l'atmosphère à la fois pour des raisons de valorisation des produits qu'ils contiennent et également pour des raisons de législation concernant la protection de l'environnement. Ces gaz sont le plus souvent soumis à une réduction permettant de transformer en soufre la majeure partie des oxydes de soufre qu'ils contiennent. Dans le cas où le procédé selon la présente invention est utilisé dans la raffinerie, il s'intègre très bien dans cette raffinerie et le dioxyde de soufre formé peut être envoyé dans une unité CLAUS (traitement des fumées), qui est pratiquement toujours présente dans cet environnement, dans laquelle il est réduit en soufre qui est ensuite éventuellement retransformé par oxydation en anhydride sulfurique.
L'exemple suivant illustre l'invention sans en limiter la portée

### Exemple

Retraitement d'un catalyseur usé constitué d'acide sulfurique imprégné sur silice et ayant été testé dans une unité pilote d'alkylation de l'isobutane par les oléfines.

### - composition du catalyseur d'alkylation à l'état neuf

Le catalyseur d'alkylation à l'état neuf, est constitué de 41,7% en poids d'une silice de diamètre moyen des particules égal à 115 µm et de 58,3% en poids d'acide sulfurique anhydre.

### - utilisation du catalyseur en alkylation de l'isobutane

Pour alkyler l'isobutane par le butène-1, on utilise un réacteur tubulaire de diamètre égal à 7 cm et de hauteur égale à 15 cm. Ce réacteur, qui contient 200 g du catalyseur dont la composition est donnée ci-dessus, est alimenté par le bas par une phase liquide dont la vitesse linéaire, dans le réacteur est égale à 0,36 cm/sec, vitesse suffisante pour assurer la fluidisation du catalyseur.

Afin de simuler le recyclage de l'isobutane de la zone de séparation isobutane/alkylat vers l'entrée du réacteur, on utilise comme charge un mélange liquide d'isobutane et de butène-1 contenant 8,3% en poids de butène-1.

Le débit volumique de la charge est égal à 102 ml/h.

La majeure partie de l'effluent liquide sortant du réacteur est recyclée vers l'entrée dudit réacteur après mélange avec la charge. Pour cela on utilise un débit volumique de recycle égal à 50 l/h. On soutire en continu de l'unité à un débit voisin de 100 ml/h une phase liquide contenant de l'isobutane, du butène-1 non converti et l'alkylat produit.

La température dans le réacteur est régulée à -3°C, la pression est de 5 bar.

Après 15 jours de test, on décharge le catalyseur pour le régénérer en utilisant le procédé décrit dans la présente invention.

### - régénération du catalyseur usé

200 g de catalyseur usé, qui se présentent sous la forme d'une poudre sèche, sont calcinés, sous un débit de 1,9 l/h/g d'air sec, à une température de 290°C pendant 5 heures, les gaz sont refroidis et le liquide condensé est recueilli dans un ballon.

Après calcination, on récupère d'une part une fraction liquide dont la masse est égale à 87,54 g, et d'autre part une poudre séche dont la masse est égale à 84,7 g. La masse correspondant aux gaz non condensés est de 27,76 g.

Le liquide recueilli est de l'acide sulfurique de titre égal à environ 93% poids, le résidu carboné présent sur la silice (environ 1,1% pois de carbone) peut être totalement éliminé par calcination supplémentaire sous air à 550°C pendant 4 heures.

Le procédé selon la présente invention permet de récupérer environ 75% de l'acide sulfurique initialement présent dans le catalyseur.

## Revendications

1. Procédé de purification d'un catalyseur d'alkylation impur à base d'acide sulfurique déposé sur une silice, par élimination des matières organiques hydrocarbonées qu'il contient, ledit catalyseur renfermant au moins 0,1% en poids, exprimé en atomes de carbone, de matières organiques sous forme libre ou combinée, procédé caractérisé en ce que ledit catalyseur est calciné sous débit d'un gaz à une température comprise entre 100 et 400°C.

2. Procédé selon la revendication 1 dans lequel à l'issue de l'étape de calcination (appelée ci-après première étape de calcination), on poursuit une calcination au cours d'une seconde étape de calcination sous débit d'un gaz, à une température comprise entre 400 et 600°C de façon à éliminer les dépôts hydrocarbonés encore présents sur le catalyseur.

3. Procédé selon l'une des revendications 1 et 2 dans lequel au cours de la première étape, le catalyseur est calciné à une température comprise entre 100 et 350°C.

4. Procédé selon la revendication 3 dans lequel la calcination est réalisée entre 170 et 330°C.

5. Procédé selon l'une des revendications 1 à 4, dans lequel au cours de la première étape, la durée de calcination est comprise entre quelques minutes et 8 heures.

6. Procédé selon l'une des revendications 1 à 5 dans lequel au cours de la deuxième étape, la calcination est effectuée entre 450 et 550°C.

7. Procédé selon l'une des revendications 1 à 6 dans lequel le gaz utilisé pour l'étape ou les étapes de calcination est un gaz contenant de l'oxygène moléculaire.

8. Procédé selon l'une des revendications 1 à 7 dans lequel les gaz formés au cours de l'étape ou les étapes de calcination, sont soumis à une réduction, avec transformation en soufre de la majeure partie des oxydes de soufre qu'ils contiennent.
